# EUROPÄISCHE PATENTANMELDUNG

(11) **EP 2 039 684 A1**
(43) Veröffentlichungstag der Anmeldung: **25.03.2009**
(21) Anmeldenummer: 07116641.7
(22) Anmeldetag: 18.09.2007
(51) Int. Cl.: C07D 213/61

(54) **Verfahren zur Herstellung von 2,2-Difluorethylamin-Derivaten durch Imin-Hydrierung**

(71) Anmelder: Bayer CropScience AG, 40789 Monheim (DE)
(72) Erfinder: Die Erfindernennung liegt noch nicht vor

(57) **Zusammenfassung**

Verfahren zur Herstellung von 2,2-Difluorethylamin-Derivaten, wobei man Verbindungen der allgemeinen Formel (IV) zu den entsprechenden 2,2-Difluorethylamin-Derivaten der allgemeinen Formel (III) hydriert, wobei der Rest A die in der Beschreibung angegebene Bedeutung aufweist:

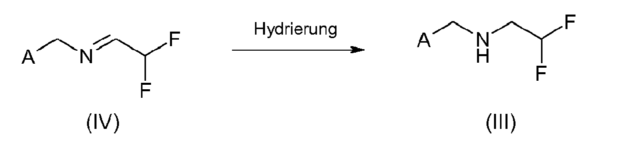

## Beschreibung

Die vorliegende Erfindung betrifft ein Verfahren zur Herstellung von 2,2-Difluorethanamin-Derivaten ausgehend von 2,2-Difluorethylimin-Derivaten. Weiterer Gegenstand der vorliegenden Erfindung sind die in diesem erfindungsgemäßen Verfahren als Ausgangsverbindungen eingesetzten 2,2-Difluoreethylimin-Derivate, deren Herstellung sowie deren Verwendung zur Herstellung von 2,2-Difluorethylamin-Derivaten.

Derivate von 2,2-Difluorethylaminen sind wichtige Zwischenprodukte zur Herstellung von agrochemischen Wirkstoffen. Entsprechende 2,2-Difluorethylamin-Derivate können beispielsweise in der Synthese von als insektizid wirksamen Enaminocarbonylverbindungen, beispielsweise von 4-Aminobut-2-enolidverbindungen, eingesetzt werden. Enaminocarbonylverbindungen, welche 2,2-Difluorethylaminobausteine umfassen, sind beispielsweise aus den nicht vorveröffentlichten deutschen Patentanmeldungen DE 10 2006 015 467 A und DE 10 2006 015 468 A bekannt.

Aus der DE 10 2006 015 467 A ist bekannt, dass man 2,2-Difluorethylamin-Derivate, wie beispielsweise die Verbindung der unten stehenden Formel (IIIa), durch Alkylierung des Amins der Formel (Ia) mit gegebenenfalls substituiertem Chlormethylpyridin der Formel (IIa) herstellen kann (Schema 1 der DE 10 2006 015 467 A; vgl. Herstellung von Ausgangsverbindungen; Verbindungen der Formel (III); III-1: N-[(6-Chlorpyridin-3-yl)methyl]-2,2difluorethyl-1-amin).

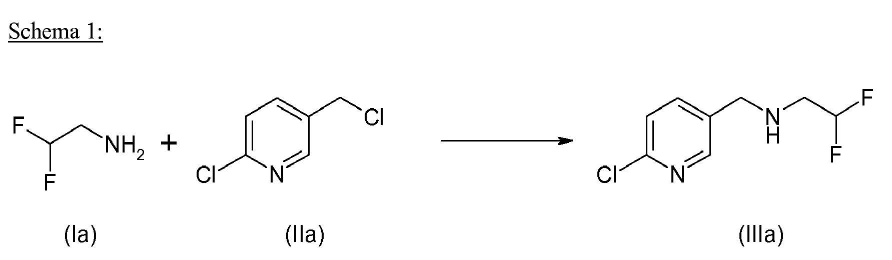

Nachteilig bei diesem Verfahren ist die geringe Ausbeute von 53 %, welche durch die mögliche Mehrfachalkylierung des Stickstoffatoms bedingt ist. Dieser Anteil der Mehrfachalkylierung kann nur durch den Einsatz eines großen Überschusses an Amin reduziert werden, was aber bei einem kostenintensiven Amin unwirtschaftlich ist.

Ausgehend von diesem Stand der Technik ergibt sich als Aufgabe der vorliegenden Erfindung, ein Verfahren zur Herstellung von 2,2-Difluorethylamin-Derivaten bereitzustellen, welches vorzugsweise einfach und kostengünstig durchzuführen ist. Die mit diesem angestrebten Verfahren erhältlichen 2,2-Difluorethylamin-Derivate sollen dabei vorzugsweise mit hoher Ausbeute und hoher Reinheit erhalten werden. Insbesondere soll das angestrebte Verfahren den Erhalt der gewünschten Zielverbindungen ohne die Notwendigkeit komplexer Aufreinigungsmethoden ermöglichen.

Gelöst wird diese Aufgabe durch ein Verfahren zur Herstellung von 2,2-Difluorethylamin-Derivaten.

Das erfindungsgemäße Verfahren ist dadurch gekennzeichnet, dass man 2,2-Difluorethylimin-Derivate der allgemeinen Formel (IV) zu den entsprechenden Zielverbindungen der allgemeinen Formel (III) gemäß nachfolgendem Schema 2 hydriert:

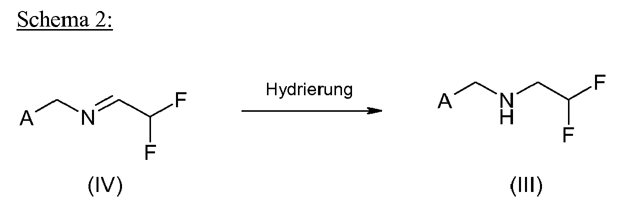

Erfindungsgemäß ist somit vorgesehen, dass die gewünschten 2,2-Difluorethylamin-Derivate der allgemeinen Formel (III) durch eine Hydrierung der entsprechenden 2,2-Difluorethylimin-Derivate der allgemeinen Formel (IV) hergestellt werden. Die gewünschten 2,2-Difluorethylamin-Derivate der allgemeinen Formel (III) werden unter den erfindungsgemäßen und weiter unten näher spezifizierten bevorzugten Reaktionsbedingungen mit guten Ausbeuten in hoher Reinheit erhalten, womit das erfindungsgemäße Verfahren die oben genannten Nachteile überwindet. Die gewünschten Verbindungen werden dabei in einer Reinheit erhalten, welche eine umfangreiche Aufarbeitung des unmittelbaren Reaktionsprodukts im Allgemeinen nicht erforderlich macht. Gegenüber dem aus dem Stand der Technik bekannten Verfahren, welches von einem zu alkylierenden Amin gemäß Schema 1 ausgeht, können die Ausbeuten durch das erfindungsgemäße Verfahren verbessert werden. Darüber hinaus ist die mit dem erfindungsgemäßen Verfahren erzielte Reinheit der gewünschten Zielverbindung größer, da keine Mehrfachalkylierung stattfindet.

Im Rahmen der vorliegenden Erfindung wird unter einem Derivat ein von dem bezeichneten organischen Grundgerüst (Baustein) abgeleiteter Stoff ähnlicher Struktur bezeichnet, d.h. unter einem 2,2-Difluorethylamin-Derivat wird beispielsweise eine Verbindung verstanden, welche einen 2,2-Difluorethylamin-Baustein umfasst.

### In den oben genannten allgemeinen Formel (III) und (IV) weist der Rest A die folgende Bedeutung auf:

- Pyrid-2-yl oder Pyrid-4-yl steht oder für Pyrid-3-yl, welches gegebenenfalls in 6-Position substituiert ist durch Fluor, Chlor, Brom, Methyl, Trifluormethyl oder Trifluormethoxy oder für Pyridazin-3-yl, welches gegebenenfalls in 6-Position substituiert ist durch Chlor oder Methyl oder für Pyrazin-3-yl oder für 2-Chlor-pyrazin-5-yl oder für 1,3-Thiazol-5-yl, welches gegebenenfalls in 2-Position substituiert ist durch Chlor oder Methyl, oder
- Pyrimidinyl, Pyrazolyl, Thiophenyl, Oxazolyl, Isoxazolyl, 1,2,4-Oxadiazolyl, Isothiazolyl, 1,2,4-Triazolyl oder 1,2,5-Thiadiazolyl steht, welcher gegebenenfalls durch Fluor, Chlor, Brom, Cyano, Nitro, C₁-C₄-Alkyl (welches gegebenenfalls durch Fluor und/oder Chlor substituiert ist), C₁-C₃-Alkylthio (welches gegebenenfalls durch Fluor und/oder Chlor substituiert ist), oder C₁-C₃-Alkylsulfonyl (welches gegebenenfalls durch Fluor und/oder Chlor substituiert ist), substituiert ist, oder
- 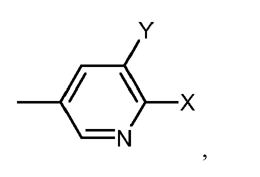 in welchem
   - X: für Halogen, Alkyl oder Halogenalkyl steht und
   - Y: für Halogen, Alkyl, Halogenalkyl, Halogenalkoxy, Azido oder Cyan steht.

Bevorzugte, besonders bevorzugte und ganz besonders bevorzugte Bedeutungen des in den oben erwähnten allgemeinen Formeln (III) und (IV) aufgeführten Restes A werden im Folgenden erläutert.
- A: wird bevorzugt ausgewählt aus der Gruppe, bestehend aus 6-Fluor-pyrid-3-yl, 6-Chlor-pyrid-3-yl, 6-Brom-pyrid-3-yl, 6-Methyl-pyrid-3-yl, 6-Trifluormethyl-pyrid-3-yl, 6-Trifluormethoxypyrid-3-yl, 6-Chlor-1,4-pyridazin-3-yl, 6-Methyl-1,4-pyridazin-3-yl, 2-Chlor-1,3-thiazol-5-yl oder 2-Methyl-1,3-thiazol-5-yl, 2-Chlor-pyrimidin-5-yl, 2-Trifluormethyl-pyrimidin-5-yl, 5,6-Difluor-pyrid-3-yl, 5-Chlor-6-fluor-pyrid-3-yl, 5-Brom-6-fluor-pyrid-3-yl, 5-Iod-6-fluor-pyrid-3-yl, 5-Fluor-6-chlor-pyrid-3-yl, 5,6-dichlor-pyrid-3-yl, 5-Brom-6-chlor-pyrid-3-yl, 5-Iod-6-chlor-pyrid-3-yl, 5-Fluor-6-brom-pyrid-3-yl, 5-Chlor-6-brom-pyrid-3-yl, 5,6-Dibrom-pyrid-3-yl, 5-Fluor-6-iod-pyrid-3-yl, 5-Chlor-6-iod-pyrid-3-yl, 5-Brom-6-iod-pyrid-3-yl, 5-Methyl-6-fluor-pyrid-3-yl, 5-Methyl-6-chlor-pyrid-3-yl, 5-Methyl-6-brom-pyrid-3-yl, 5-Methyl-6-iod-pyrid-3-yl, 5-Difluormethyl-6-fluor-pyrid-3-yl, 5-Difluormethyl-6-chlor-pyrid-3-yl, 5-Difluormethyl-6-brom-pyrid-3-yl und 5-Difluormethyl-6-iod-pyrid-3-yl.
- A: wird besonders bevorzugt ausgewählt aus der Gruppe, bestehend aus 6-Fluor-pyrid-3-yl, 6-Chlor-pyrid-3-yl, 6-Brompyrid-3-yl, 6-Chlor-1,4-pyridazin-3-yl, 2-Chlor-1,3-thiazol-5-yl, 2-Chlor-pyrimidin-5-yl, 5-Fluor-6-chlor-pyrid-3-yl, 5,6-Dichlor-pyrid-3-yl, 5-Brom-6-chlor-pyrid-3-yl, 5-Fluor-6-brom-pyrid-3-yl, 5-Chlor-6-brom-pyrid-3-yl, 5,6-Dibrom-pyrid-3-yl, 5-Methyl-6-chlor-pyrid-3-yl, 5-Chlor-6-iod-pyrid-3-yl und 5-Difluormethyl-6-chlor-pyrid-3-yl.
- A: wird ganz besonders bevorzugt ausgewählt aus der Gruppe, bestehend aus 6-Chlor-pyrid-3-yl, 6-Brom-pyrid-3-yl, 6-Chlor-1,4-pyridazin-3-yl, 2-Chlor-1,3-thiazol-5-yl, 5-Fluor-6-chlor-pyrid-3-yl und 5-Fluor-6-brom-pyrid-3-yl.

Unter dem Begriff "Alkyl", entweder in Alleinstellung oder aber in Kombination mit weiteren Begriffen, wie beispielsweise Halogenalkyl, wird im Rahmen der vorliegenden Erfindung ein Rest einer gesättigten, aliphatischen Kohlenwasserstoffgruppe mit 1 bis 12 Kohlenstoffatomen verstanden, die verzweigt oder unverzweigt sein kann. Beispiele für C₁-C₁₂-Alkylreste sind Methyl, Ethyl, n-Propyl, iso-Propyl, n-Butyl, iso-Butyl, sek.-Butyl, tert.-Butyl, n-Pentyl, iso-Pentyl, Neopentyl, tert.-Pentyl, 1-Methylbutyl, 2-Methylbutyl, 1-Ethylpropyl, 1,2-Dimethylpropyl, Hexyl n-Heptyl, n-Octyl, n-Nonyl, n-Decyl, n-Undecyl und n-Dodecyl. Von diesen Alyklresten sind C₁-C₆-Alkylreste besonders bevorzugt. Insbesondere bevorzugt sind C₁-C₄-Alkylreste.

Unter dem Begriff "Aryl" wird erfindungsgemäß ein aromatischer Rest mit 6 bis 14 Kohlenstoffatomen, vorzugsweise Phenyl, verstanden.

Unter dem Begriff "Arylalkyl" wird eine Kombination von erfindungsgemäß definierten Resten "Aryl" und "Alkyl" verstanden, wobei der Rest im Allgemeinen über die Alkylgrupe gebunden wird, Beispiele hierfür sind Benzyl, Phenylethyl oder α-Methylbenzyl, wobei Benzyl besonders bevorzugt ist.

Im Rahmen der vorliegenden Erfindung werden durch Halogen substituierte Reste, beispielsweise Halogenalkyl, einfach oder mehrfach bis zur maximal möglichen Substituentenzahl halogenierte Reste verstanden. Bei mehrfacher Halogenierung können die Halogenatome gleich oder verschieden sein. Halogen steht dabei für Fluor, Chlor, Brom oder Iod, insbesondere für Fluor, Chlor oder Brom.

Unter dem Begriff "Alkoxy", entweder in Alleinstellung oder aber in Kombination mit weiteren Begriffen, wie beispielsweise Halogenalkoxy, wird vorliegend ein Rest O-Alkyl verstanden, wobei der Begriff "Alkyl" die oben stehende Bedeutung aufweist.

Gegebenenfalls substituierte Reste können einfach oder mehrfach substituiert sein, wobei bei einer Mehrfachsubstitutionen die Substituenten gleich oder verschieden sein können.

Die Hydrierung der 2,2-Difluorethylimin-Derivate der allgemeinen Formel (IV) zu den entsprechenden Aminen der allgemeinen Formel (III) kann mit dem Fachmann an sich bekannten Reduktionsmitteln erfolgen. Beispielsweise ist es möglich, die Reduktion mit
- komplexen Hydriden,
- nichtkomplexen Metall- oder Halbmetallhydriden,
- Na/EtOH, oder
- durch katalytische Hydrierung
durchzuführen.

Unter komplexen Hydriden versteht man im Allgemeinen geladene Metallkomplexe, die wenigstens einen Hydrid-Liganden enthalten. Beispiele hierfür sind Lithiumaluminiumhydrid (LiAlH₄), LiAlH(O-tert-butyl)₃, LiAlH(O-methyl)₃, NaAlEt₂H₂, Natriumborhydrid (NaBH₄) und dergleichen. Beispiele für nichtkomplexe Metall- und Halbmetallhydride sind AlH₃, DIBAL-H (AlH(isobutyl)₂) und dergleichen. Hiervon ist die Verwendung von Natriumborhydrid (NaBH₄) besonders bevorzugt. Die Umsetzung mit den komplexen Metallhydriden bzw. den nichtkomplexe Metall- oder Halbmetallhydriden kann im Vakuum, bei Normaldruck oder unter Überdruck und bei Temperaturen von -30 bis 150 °C, vorzugsweise -10 bis 60 °C, durchgeführt werden.

Wenn eine katalytische Hydrierung zur Reduktion der Verbindung der allgemeinen Formel (IV) angewendet wird, kann als Katalysator ein beliebiger Hydrierkatalysator verwendet werden. Geeignete Katalysatoren enthalten gegebenenfalls ein oder mehrere Metalle der Gruppen 8 - 10 des Periodensystems auf einem beliebigen üblichen anorganischen Träger. In Frage kommen beispielsweise Edelmetallkatalysatoren, wie Rutheniumkatalysatoren, Palladiumkatalysatoren, Platinkatalysatoren und Rhodiumkatalysatoren, Raney-Nickel-Katalysatoren und Lindlar-Katalysatoren. Neben diesen heterogenen Katalysatoren können jedoch auch Hydrierungen an homogenen Katalysatoren durchgeführt werden, beispielsweise an dem Wilkinson-Katalysator. Die entsprechenden Katalysatoren können auch in geträgerter Form, beispielsweise auf Kohlenstoffstoff (Kohle oder Aktivkohle), Aluminiumoxid, Siliciumdioxid, Zirkondioxid oder Titandioxid, aufgebracht, verwendet werden. Entsprechende Katalysatoren sind dem Fachmann an sich bekannt. Insbesondere bevorzugt sind Raney-Nickel-Katalysatoren.

Die katalytische Hydrierung kann unter Überdruck in einem Autoklaven oder bei Normaldruck in einer Wasserstoffgasatmosphäre durchgeführt werden. Die Wasserstoffgasatmosphäre kann dabei zusätzlich auch inerte Gase, beispielsweise Argon oder Stickstoff, enthalten. Die katalytische Hydrierung wird vorzugsweise bei einer Temperatur von 10 bis 60 °C, besonders bevorzugt bei 20 bis 40 °C durchgeführt. Der Wasserstoffdruck beträgt üblicherweise 0,1 bis 50 bar, vorzugsweise 0,1 bis 30 bar.

Weitere, für die Hydrierung von Iminen verwendete Reagenzien und Hydrierbedingungen sind in den Veröffentlichungen von Harada, in Patai, "The chemistry of the Carbon-Nitrogen Double Bond", Seiten 276 bis 293; und von Ryländer, "Catalytic Hydrogenation over Platinum Metals", Seiten 291 bis 303, Academic Press, New York, 1967 beschrieben.

Im Allgemeinen ist es vorteilhaft, das erfindungsgemäße Verfahren der Hydrierung der Imine in Gegenwart von Lösungsmitteln (Verdünnungsmitteln) durchzuführen. Lösungsmittel werden vorteilhaft in einer solchen Menge eingesetzt, dass das Reaktionsgemisch während des ganzen Verfahrens der Reduktion gut rührbar bleibt. Als Lösungsmittel zur Durchführung des erfindungsgemäßen Verfahrens kommen alle unter den Reaktionsbedingungen inerten organischen Lösungsmittel in Frage, wobei die Art des verwendeten Lösungsmittels von der Art der Reduktionsdurchführung, d.h. insbesondere von der Art des Reduktionsmittels, abhängt.

Als Beispiele sind zu nennen: Halogenkohlenwasserstoffe, insbesondere Chlorkohlenwasserstoffe, wie Tetrachlorethylen, Tetrachlorethan, Dichlorpropan, Methylenchlorid, Dichlorbutan, Chloroform, Tetrachlorkohlenstoff, Trichlorethan, Trichlorethylen, Pentachlorethan, Difluorbenzol, 1,2-Dichlorethan, Chlorbenzol, Brombenzol, Dichlorbenzol, Chlortoluol, Trichlorbenzol; Alkohole wie Methanol, Ethanol, Isopropanol, Butanol; Ether, wie Ethylpropylether, Methyl-tert-butylether, *n-*Butylether, Anisol, Phenetol, Cyclohexylmethylether, Dimethylether, Diethylether, Dimethylglycol Diphenylether, Diproplether, Diisopropylether, Di-*n*-butylether, Diisobutylether, Diisoamylether, Ethylenglycoldimethylether, Isopropylethylether, Methyl-tert-butylether, Tetrahydrofuran, Methyl-Tetrahydrofuran, Dioxan, Dichlordiethylether und Polyether des Ethylenoxids und/oder Propylenoxids; Amine wie Trimethyl-, Triethyl-, Tripropyl-, Tributylamin, *N*-Methyhnorpholin, Pyridin, alkylierte Pyridine und Tetramethylendiamin; aliphatische, cycloaliphatische oder aromatische Kohlenwasserstoffe wie Pentan, n-Hexan, n-Heptan, n-Oktan, Nonan und technische Kohlenwasserstoffe welche durch Fluor- und Chloratome substituiert sein können, wie Methylenchlorid, Dichlormethan, Trichlormethan, Tetrachlorkohlenstoff, Fluorbenzol, Chlorbenzol oder Dichlorbenzol; beispielsweise sogenannte White Spirits mit Komponenten mit Siedepunkten im Bereich beispielsweise von 40°C bis 250°C, Cymol, Benzinfraktionen innerhalb eines Siedeinterwalles von 70°C bis 190°C, Cyclohexan, Methylcyclohexan, Petrolether, Ligroin, Octan, Benzol, Toluol, Chlorbenzol, Brombenzol, Nitrobenzol, Xylol; Ester wie Methyl-, Ethyl-, Butyl-, Isobutylacetat, sowie Dimethyl-, Dibutyl-, Ethylencarbonat; und aliphatische Alkohole, wie Methanol, Ethanol, n-Propanol und iso-Propanol und n-Butanol.

Von den zuvor genannten Lösungsmitteln sind Alkohole, insbesondere Methanol und Ethanol, speziell Methanol, bevorzugt.

Die in der erfindungsgemäßen Umsetzung verwendeten Lösungsmittelmengen können in einem weiten Bereich variiert werden. Im Allgemeinen werden Lösungsmittelmengen im Bereich von der 1-fachen bis 50-fachen Lösungsmittelmenge, besonders bevorzugt von der 2-fachen bis 40-fachen Lösungsmittelmenge, insbesondere von der 2-fachen bis 30-fachen Lösungsmittelmenge, jeweils bezogen auf das eingesetzte 2,2-Difluorethylimin der allgemeinen Formel (IV), verwendet.

Darüber hinaus ist insbesondere die Kombination von Natriumborhydrid (NaBH₄) als Hydriermittel in Kombination mit Alkoholen, speziell Methanol, als Lösungsmittel bevorzugt.

Die erfindungsgemäße Umsetzung kann mit diesem System aus Natriumborhydrid (NaBH₄) und Methanol insbesondere wie folgt durchgeführt werden: Das Imin wird in dem Alkohol vorgelegt und das Natriumborhydrid wird unter Kühlung in Portionen zugesetzt. Anschließend wird bei einer Temperatur von 30 bis 50 °C gerührt und dann etwa 1 bis 3 Äquivalente an Wasser, bezogen auf die Alkoholmenge, zugesetzt. Anschließend wird auf übliche Weise mit einem organischen Lösungsmittel extrahiert.

Die Hydrierung erfolgt im Allgemeinen unter solchen Reaktionsbedingungen (Druck, Temperatur, Stöchiometrie etc.), unter denen die Imin-Gruppe zu einer gesättigten Gruppen hydriert wird, gleichzeitig jedoch die übrigen im Molekül vorhandenen funktionellen Gruppen unverändert bleiben.

Die Aufarbeitung (Reinigung) und Isolierung der hydrierten Imine kann beispielsweise durch Kristallisation und / oder Destillation erfolgen.

Die vorliegende Erfindung betrifft darüber hinaus auch die Verwendung der Verbindungen der allgemeinen Formel (IV) zur Herstellung von Verbindungen der allgemeinen Formel (III), wie es in dem oben beschriebenen Verfahren offenbart ist.

Die für die erfindungsgemäße Umsetzung erforderlichen Verbindungen der allgemeinen Formel (IV) können dadurch erhalten werden, dass man Aldehyde der allgemeinen Formel (VI) 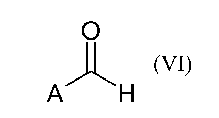 in welcher
- A: die oben genannten Bedeutungen aufweist
mit 2,2-Difluorethylamin zu einer Verbindung der allgemeinen Formel (IV) 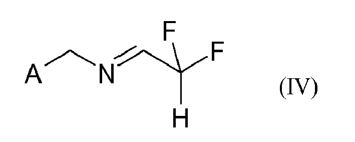 unter Kondensation umsetzt.

Das für diese Umsetzung benötigte 2,2-Difluorethylamin ist kommerziell erhältlich und kann nach literaturbekannten Verfahren hergestellt werden (J. Medicinal Chemistry (1989), 32(5), 957-961).

Die Aldehyde der Formel (II) sind kommerziell erhältlich oder können nach literaturbekannten Verfahren hergestellt werden (vgl. z.B. 6-Methyl-nicotinaldehyd: EP 0 104 876 A2; 2-Chlor-pyrazin-5-carboxaldehyd: DE 3314196 A1).

Der Umsetzung zum Erhalt der Verbindungen der allgemeinen Formel (IV) kann gegebenenfalls eine Säure als Katalysator zugesetzt werden. Beispiele hierfür sind Essigsäure, p-Toluolsulfonsäure, Trifluoressigsäure. Bevorzugt verwendet wird Essigsäure.

Werden entsprechende Katalysatoren eingesetzt, so kann deren Menge von 0,01 bis 10 Gewichtsprozent, bezogen auf das eingesetzte 2,2-Difluorethylamin, betragen.

In einer bevorzugten Ausführungsform der vorliegenden Erfindung wird das 2,2-Difluorethylamin in der Form eines Salzes eingesetzt. Dadurch kann der Zusatz an Lewis-Säure entfallen oder reduziert werden. Als Salze kommen beispielsweise das Hydrochlorid, das Acetat oder das Sulfat in Frage.

Die Umsetzung zur Herstellung der Verbindungen der allgemeinen Formel (IV) kann darüber hinaus auch so durchgeführt werden, dass das bei der Reaktion zwischen Amin und Aldehyd durch Kondensation entstehende Wasser aus dem Reaktionsgemisch entfernt wird. Dies ist beispielsweise durch Verwendung von wasserbindenden Mitteln, beispielsweise Natriumsulfat, Magnesiumsulfat oder Molekularsieb, oder durch Verwendung einer Vorrichtung zur Wasserabscheidung möglich.

Die Umsetzung zur Herstellung der Verbindungen der allgemeinen Formel (IV) kann im Allgemeinen im Vakuum, bei Normaldruck oder unter Überdruck durchgeführt werden. Die angewendeten Temperaturen können ebenfalls, in Abhängigkeit der verwendeten Substrate, variieren und sind für den Fachmann durch Routineversuche leicht zu ermitteln. Beispielsweise kann die Umsetzung zur Herstellung der Verbindungen der allgemeinen Formel (IV) bei einer Temperatur von -20 bis 200 °C, vorzugsweise 10 bis 100 °C, durchgeführt werden. Besonders bevorzugt wird die Umsetzung bei Normaldruck und Temperaturen von 10 bis 100 °C durchgeführt werden.

Die Umsetzung zur Herstellung der Imine der allgemeinen Formel (IV) kann darüber hinaus auch in Gegenwart von Lösungsmitteln (Verdünnungsmitteln) durchgeführt werden. Die Lösungsmittel werden auch in diesem Verfahrensschritt vorzugsweise in einer solchen Menge eingesetzt, dass das Reaktionsgemisch während des ganzen Verfahrens der Reduktion gut rührbar bleibt. Als Lösungsmittel zur Durchführung des erfindungsgemäßen Verfahrens zur Herstellung der 2,2-Difluorethylimin-Derivate der allgemeinen Formel (IV) kommen alle unter den Reaktionsbedingungen inerten organischen Lösungsmittel in Frage.

Als Beispiele sind zu nennen: Halogenkohlenwasserstoffe, insbesondere Chlorkohlenwasserstoffe, wie Tetrachlorethylen, Tetrachlorethan, Dichlorpropan, Methylenchlorid, Dichlorbutan, Chloroform, Tetrachlorkohlenstoff, Trichlorethan, Trichlorethylen, Pentachlorethan, Fluorbenzol, Difluorbenzol, 1,2-Dichlorethan, Chlorbenzol, Brombenzol, Dichlorbenzol, Chlortoluol, Trichlorbenzol; Alkohole wie Methanol, Ethanol, Isopropanol, Butanol; Ether, wie Ethylpropylether, Methyl-tert-butylether, *n*-Butylether, Anisol, Phenetol, Cyclohexylmethylether, Dimethylether, Diethylether, Dimethylglycol Diphenylether, Diproplether, Diisopropylether, Di-*n*-butylether, Diisobutylether, Diisoamylether, Ethylenglycoldimethylether, Isopropylethylether, Methyl-tert-butylether, Tetrahydrofuran, Dioxan, Dichlordiethylether und Polyether des Ethylenoxids und/oder Propylenoxids; Nitrokohlenwasserstoffe wie Nitromethan, Nitroethan, Nitropropan, Nitrobenzol, Chlomitrobenzol, o-Nitrotoluol; Nitrile wie Acetonitril, Methylnitril, Propionitril, Butyronitril, Isobutyronitril, Benzonitril, Phenylnitril, m-Chlorbenzonitril sowie Verbindungen wie Tetrahydrothiophendioxid und Dimethylsulfoxid, Tetramethylensulfoxid, Dipropylsulfoxid, Benzylmethylsulfoxid, Diisobutylsulfoxid, Dibutylsulfoxid, Diisoamylsulfoxid; Sulfone wie Dimethyl-, Diethyl-, Dipropyl-, Dibutyl-, Diphenyl-, Dihexyl-, Methylethyl-, Ethylpropyl-, Ethylisobutyl- und Pentamethylensulfon; aliphatische, cycloaliphatische oder aromatische Kohlenwasserstoffe wie Pentan, Hexan, Heptan, Oktan, Nonan und technische Kohlenwasserstoffe welche durch Fluor- und Chloratome substituiert sein können, wie Methylenchlorid, Dichlormethan, Trichlormethan, Tetrachlorkohlenstoff, Fluorbenzol, Chlorbenzol oder Dichlorbenzol; beispielsweise sogenannte White Spirits mit Komponenten mit Siedepunkten im Bereich beispielsweise von 40°C bis 250°C, Cymol, Benzinfraktionen innerhalb eines Siedeinterwalles von 70°C bis 190°C, Cyclohexan, Methylcyclohexan, Petrolether, Ligroin, Octan, Benzol, Toluol, Chlorbenzol, Brombenzol, Nitrobenzol und Xylol. Von den zuvor genannten Lösungsmitteln sind Xylol, Chlorbenzol, Cyclohexan und Toluol insbesondere bevorzugt.

In einer weiteren Ausführungsform kann die Umsetzung zwischen Amin und Aldehyd auch in Substanz erfolgen.

Fall die Umsetzung in einem Lösungsmittel durchgeführt wird, kann das Lösungsmittel nach dem Reaktionsende durch Abdestillieren entfernt werden. Dies kann unter Normaldruck oder erniedrigtem Druck bei Raumtemperatur oder erhöhten Temperaturen erfolgen. Die Mischung kann aber auch direkt in die Hydrierung überführt werden, was insbesondere unter wirtschaftlichen Überlegungen von Vorteil ist. In dieser Ausgestaltung des erfindungsgemäßen Verfahrens wird dann auf eine Aufarbeitung des 2,2-Difluorethylimin-Derivates verzichtet.

Weiterer Gegenstand der vorliegenden Erfindung sind darüber hinaus die Verbindungen der allgemeinen Formel (IV), welche als Zwischenprodukte bei der Herstellung der Zielverbindungen der allgemeinen Formel (III) verwendet werden:

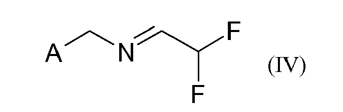

In diesen Intermediaten weist der Substituent A im Allgemeinen die folgende Bedeutung auf:
- Pyrid-2-yl oder Pyrid-4-yl steht oder für Pyrid-3-yl, welches gegebenenfalls in 6-Position substituiert ist durch Fluor, Chlor, Brom, Methyl, Trifluormethyl oder Trifluormethoxy oder für Pyridazin-3-yl, welches gegebenenfalls in 6-Position substituiert ist durch Chlor oder Methyl oder für Pyrazin-3-yl oder für 2-Chlor-pyrazin-5-yl oder für 1,3-Thiazol-5-yl, welches gegebenenfalls in 2-Position substituiert ist durch Chlor oder Methyl, oder
- Pyrimidinyl, Pyrazolyl, Thiophenyl, Oxazolyl, Isoxazolyl, 1,2,4-Oxadiazolyl, Isothiazolyl, 1,2,4-Triazolyl oder 1,2,5-Thiadiazolyl steht, welcher gegebenenfalls durch Fluor, Chlor, Brom, Cyano, Nitro, C₁-C₄-Alkyl (welches gegebenenfalls durch Fluor und/oder Chlor substituiert ist), C₁-C₃-Alkylthio (welches gegebenenfalls durch Fluor und/oder Chlor substituiert ist), oder C₁-C₃-Alkylsulfonyl (welches gegebenenfalls durch Fluor und/oder Chlor substituiert ist), substituiert ist, oder
- 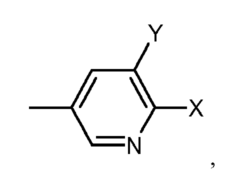 in welchem
   - X: für Halogen, Alkyl oder Halogenalkyl steht und
   - Y: für Halogen, Alkyl, Halogenalkyl, Halogenalkoxy, Azido oder Cyan steht.

Bevorzugte, besonders bevorzugte und ganz besonders bevorzugte Substituenten bzw. Bereiche des in den oben erwähnten allgemeinen Formeln (III) und (IV) aufgeführten Restes A werden im Folgenden erläutert.
- A: wird bevorzugt ausgewählt aus der Gruppe, bestehend aus 6-Fluor-pyrid-3-yl, 6-Chlor pyrid-3-yl, 6-Brom-pyrid-3-yl, 6-Methyl-pyrid-3-yl, 6-Trifluormethyl-pyrid-3-yl, 6-Trifluormethoxypyrid-3-yl, 6-Chlor-1,4-pyridazin-3-yl, 6-Methyl-1,4-pyridazin-3-yl, 2-Chlor-1,3-thiazol-5-yl oder 2-Methyl-1,3-thiazol-5-yl, 2-Chlor-pyrimidin-5-yl, 2-Trifluormethyl-pyrimidin-5-yl, 5,6-Difluor-pyrid-3-yl, 5-Chlor-6-fluor-pyrid-3-yl, 5-Brom-6-fluor-pyrid-3-yl, 5-Iod-6-fluor-pyrid-3-yl, 5-Fluor-6-chlor-pyrid-3-yl, 5,6-dichlor-pyrid-3-yl, 5-Brom-6-chlor-pyrid-3-yl, 5-Iod-6-chlor-pyrid-3-yl, 5-Fluor-6-brom-pyrid-3-yl, 5-Chlor-6-brom-pyrid-3-yl, 5,6-Dibrom-pyrid-3-yl, 5-Fluor-6-iod-pyrid-3-yl, 5-Chlor-6-iod pyrid-3-yl, 5-Brom-6-iod-pyrid-3-yl, 5-Methyl-6-fluor-pyrid-3-yl, 5-Methyl-6-chlor-pyrid-3-yl, 5-Methyl-6-brom-pyrid-3-yl, 5-Methyl-6-iod-pyrid-3-yl, 5-Difluormethyl-6-fluor-pyrid-3-yl, 5-Difluormethyl-6-chlor-pyrid-3-yl, 5-Difluormethyl-6-brom-pyrid-3-yl und 5-Difluormethyl-6-iod-pyrid-3-yl.
- A: wird besonders bevorzugt ausgewählt aus der Gruppe, bestehend aus 6-Fluor-pyrid-3-yl, 6-Chlor-pyrid-3-yl, 6-Brompyrid-3-yl, 6-Chlor-1,4-pyridazin-3-yl, 2-Chlor-1,3-thiazol-5-yl, 2-Chlor-pyrimidin-5-yl, 5-Fluor-6-chlor-pyrid-3-yl, 5,6-Dichlor-pyrid-3-yl, 5-Brom-6-chlor pyrid-3-yl, 5-Fluor-6-brom-pyrid-3-yl, 5-Chlor-6-brom-pyrid-3-yl, 5,6-Dibrom-pyrid-3-yl, 5-Methyl-6-chlor-pyrid-3-yl, 5-Chlor-6-iod-pyrid-3-yl und 5-Difluormethyl-6-chlor-pyrid-3-yl.
- A: wird ganz besonders bevorzugt ausgewählt aus der Gruppe, bestehend aus 6-Chlor-pyrid-3-yl, 6-Brom-pyrid-3-yl, 6-Chlor-1,4-pyridazin-3-yl, 2-Chlor-1,3-thiazol-5-yl, 5-Fluor-6-chlor-pyrid-3-yl und 5-Fluor-6-brom-pyrid-3-yl.

Die Verbindungen der allgemeinen Formel (IV) können als Edukt zur Herstellung der 2,2-Difluorethylamin-Derivate der allgemeinen Formel (III) verwendet werden.

Ausgehend von den Verbindungen der allgemeinen Formel (III), welche durch das erfindungsgemäße Verfahren erhalten werden, können insektizid wirksame Enaminocarbonylverbindungen, welche 2,2-Difluorethylaminobausteine umfassen und beispielsweise in den nicht vorveröffentlichten deutschen Patentanmeldungen DE 10 2006 015 467 A und DE 10 2006 015 468 A beschrieben sind, hergestellt werden.

Zu diesem Zweck werden die Verbindungen der allgemeinen Formel (III) 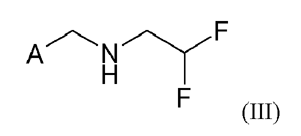 an dem sekundären Aminstickstoff beispielsweise durch Umsetzung mit Tetronsäure oder Derivaten davon alkenyliert. Eine entsprechende Umsetzung ist in der Schema I der DE 10 2006 015 467 näher beschrieben und führt unmittelbar zu den insektizid wirksamen Enaminocarbonylverbindungen.

Die vorliegende Erfindung wird anhand der nachfolgenden Beispiele näher erläutert, wobei die Beispiele nicht in die Erfindung einschränkender Weise zu interpretieren sind.

### Herstellungsbeispiele:

### Beispiel 1

Zu einer Lösung von 74,34 g 6-Chlorpyridin-3-carbaldehyd in 46 g Toluol werden 41,8 g 2,2-Difluorethylamin bei Raumtemperatur zugesetzt. Die Reaktionsmischung wird 2 Stunden bei Raumtemperatur gerührt. Anschließend wird 120 g wasserfreies Magnesiumsulfat zugesetzt und weitere 5 Stunden bei 50°C gerührt. Die Reaktionsmischung wird auf Raumtemperatur abgekühlt, filtriert und der Filterrückstand mit Toluol gewaschen. Das Lösungsmittel wird im Vakuum entfernt und man erhält 100,2 g N-[(6-Chlorpyridin-3-yl)methyliden)]-2,2-difluorethylamin in einer Reinheit von 95,5 % (dies entspricht 94 % Ausbeute).
¹H-NMR (CDCl₃, 298K) δ: 4,05 m (2H), 6,5 t (1 H, CHF₂), 7,63 d (1H), 8,20 d (1H), 8,56 s (1H), 8,75 d( 1H)

### Beispiel 2

Zu einer Lösung von 74 g N-[(6-Chlorpyridin-3-yl)methyliden)]-2,2-difluorethylamin (aus Beispiel 1) in 343 g Ethanol werden 5 g Raney-Nickel-Katalysator gegeben und bei Raumtemperatur mit 20 bar Wasserstoff 24 h hydriert. Der Katalysator wird abfiltriert, der Rückstand mit 100 ml Ethanol gewaschen und das Lösungsmittel im Vakuum entfernt. Man erhält 71,4 g -[(6-Chlorpyridin-3-yl)methyl)]-2,2-difluorethylamin in einer Reinheit von 95 % (dies entspricht 94 % Ausbeute).
NMR(d-DMSO): 1H(s, 8,35 ppm); 1H (dd,7,8 ppm); 1H (d,7,46 ppm); 1 H (tt, 6,02 ppm); 2 H (s, 3,8 ppm); 2 H (td, 2,9 ppm)

### Beispiel 3

Zu einer Lösung von 5 g N-[(6-Chlorpyridin-3-yl)methyliden)]-2,2-difluorethylamin in 23 g Ethanol werden 1,1 g Natriumborhydrid portionsweise zugesetzt und bei Raumtemperatur gerührt. Anschließend wird kurz auf 50 °C erhitzt und dann auf 100 ml Wasser gegossen. Die Mischung wird zweimal mit je 100 ml Methylenchlorid extrahiert und die vereinigten organischen Phasen im Vakuum eingeengt. Man erhält 4,5 g -[(6-Chlorpyridin-3-yl)methyl)]-2,2-difluorethylamin in einer Reinheit von 93 % (dies entspricht 86 % Ausbeute).
NMR-Daten: siehe Beispiel 2

## Patentansprüche

1. Verfahren zur Herstellung von 2,2-Difluorethylamin-Derivaten, **dadurch gekennzeichnet, dass** man 2,2-Difluorethylimin-Derivate der allgemeinen Formel (IV) zu den entsprechenden 2,2-Difluorethylamin-Derivaten der allgemeinen Formel (III) hydriert: 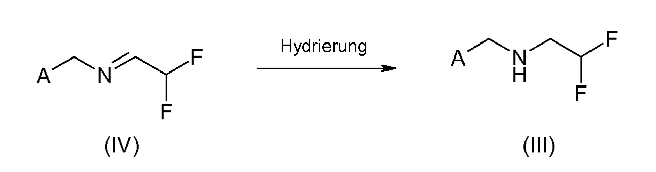 wobei der Rest A in den allgemeinen Formel (III) und (IV) die folgende Bedeutung aufweist:
o Pyrid-2-yl oder Pyrid-4-yl steht oder für Pyrid-3-yl, welches gegebenenfalls in 6-Position substituiert ist durch Fluor, Chlor, Brom, Methyl, Trifluormethyl oder Trifluormethoxy oder für Pyridazin-3-yl, welches gegebenenfalls in 6-Position substituiert ist durch Chlor oder Methyl oder für Pyrazin-3-yl oder für 2-Chlor-pyrazin-5-yl oder für 1,3-Thiazol-5-yl, welches gegebenenfalls in 2-Position substituiert ist durch Chlor oder Methyl, oder
o Pyrimidinyl, Pyrazolyl, Thiophenyl, Oxazolyl, Isoxazolyl, 1,2,4-Oxadiazolyl, Isothiazolyl, 1,2,4-Triazolyl oder 1,2,5-Thiadiazolyl steht, welcher gegebenenfalls durch Fluor, Chlor, Brom, Cyano, Nitro, C₁-C₄-Alkyl (welches gegebenenfalls durch Fluor und/oder Chlor substituiert ist), C₁-C₃-Alkylthio (welches gegebenenfalls durch Fluor und/oder Chlor substituiert ist), oder C₁-C₃-Alkylsulfonyl (welches gegebenenfalls durch Fluor und/oder Chlor substituiert ist), substituiert ist, oder
o 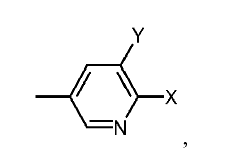 in welchem
X für Halogen, Alkyl oder Halogenalkyl steht und
Y für Halogen, Alkyl, Halogenalkyl, Halogenalkoxy, Azido oder Cyan steht.

2. Verfahren nach Anspruch 1, **dadurch gekennzeichnet, dass** die Hydrierung mit komplexen Hydriden, nichtkomplexen Metall- oder Halbmetallhydriden, Na/EtOH oder durch katalytische Hydrierung durchgeführt wird.

3. Verfahren zur Herstellung von Verbindungen der allgemeinen Formel (IV) 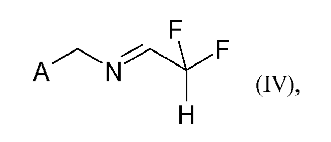 **dadurch gekennzeichnet, dass** man Verbindungen der Aldehyde der allgemeinen Formel (VI) 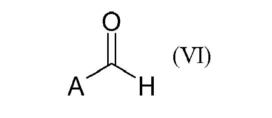 in welcher
A die oben genannten Bedeutungen aufweist
mit 2,2-Difluorethylamin umsetzt.

4. Verfahren nach Anspruch 1 oder 2, **dadurch gekennzeichnet, dass** die in dem Verfahren gemäß Anspruch 3 erhaltenen Verbindungen der allgemeinen Formel (IV) als Ausgangsverbindungen eingesetzt werden.

5. Verbindungen der allgemeinen Formel (IV) 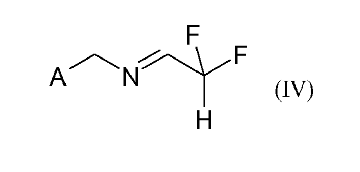 in welchen der Rest A die folgende Bedeutung aufweist:
o Pyrid-2-yl oder Pyrid-4-yl steht oder für Pyrid-3-yl, welches gegebenenfalls in 6-Position substituiert ist durch Fluor, Chlor, Brom, Methyl, Trifluormethyl oder Trifluormethoxy oder für Pyridazin-3-yl, welches gegebenenfalls in 6-Position substituiert ist durch Chlor oder Methyl oder für Pyrazin-3-yl oder für 2-Chlor-pyrazin-5-yl oder für 1,3-Thiazol-5-yl, welches gegebenenfalls in 2-Position substituiert ist durch Chlor oder Methyl, oder
o Pyrimidinyl, Pyrazolyl, Thiophenyl, Oxazolyl, Isoxazolyl, 1,2,4-Oxadiazolyl, Isothiazolyl, 1,2,4-Triazolyl oder 1,2,5-Thiadiazolyl steht, welcher gegebenenfalls durch Fluor, Chlor, Brom, Cyano, Nitro, C₁-C₄-Alkyl (welches gegebenenfalls durch Fluor und/oder Chlor substituiert ist), C₁-C₃-Alkylthio (welches gegebenenfalls durch Fluor und/oder Chlor substituiert ist), oder C₁-C₃-Alkylsulfonyl (welches gegebenenfalls durch Fluor und/oder Chlor substituiert ist), substituiert ist, oder
o 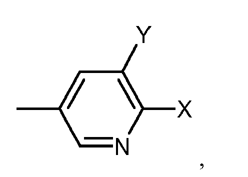 in welchem
X für Halogen, Alkyl oder Halogenalkyl steht und
Y für Halogen, Alkyl, Halogenalkyl, Halogenalkoxy, Azido oder Cyan steht.

6. Verwendung der Verbindungen der allgemeinen Formel (IV) gemäß Anspruch 5 zur Herstellung von Verbindungen der allgemeinen Formel (III) nach dem Verfahren gemäß Anspruch 1 oder 2.
